Europäisches Patentamt

(19) European Patent Office

Office européen des brevets

(11) Publication number: **0 176 277**
**A2**

(12) **EUROPEAN PATENT APPLICATION**

(21) Application number: **85306405.3**

(22) Date of filing: **10.09.85**

(51) Int. Cl.⁴: **A 63 B 21/00**
**//A61B5/22, A61B5/08**

(30) Priority: **25.09.84 JP 198636/84**

(43) Date of publication of application:
**02.04.86 Bulletin 86/14**

(84) Designated Contracting States:
**CH DE FR GB IT LI**

(71) Applicant: **KABUSHIKI KAISHA SILVER MEDICAL**
**12-5, Hongo 3-chome**
**Bunkyo-ku Tokyo(JP)**

(72) Inventor: **Fujiwara, Takayuki**
**7-16, Matsuyama 2-chome**
**Kiyose-shi Tokyo(JP)**

(74) Representative: **Thomson, Paul Anthony et al,**
**Potts, Kerr & Co. 15, Hamilton Square**
**Birkenhead Merseyside L41 6BR(GB)**

(54) Optimum exercise loading apparatus.

(57) An optimum exercise loading apparatus is disclosed which is capable of automatically and real-timely determining optimum exercise load for an individual patient and permitting the patient to carry out loading exercise under the optimum conditions, to thereby accomplish both the satisfied risk management and positive exercise cure of the patient. The optimum exercise loading apparatus comprises a load section (A) for causing a patient to carry out exercise under load, a measuring and analyzing section (B) for carrying out the measuring and analyzing of oxygen intake or $VO_2$ of the patient during the exercise, and a control section (C) for carrying out the comparison between a measured value of $VO_2$ and a target value thereof to judge the propriety of load applied to the patient to carry out the feedback control of the load, to thereby place the patient under appropriate load.

./...

EP 0 176 277 A2

# FIG. 1

OPTIMUM EXERCISE LOADING APPARATUS

BACKGROUND OF THE INVENTION

Field of the Invention

This invention relates to an optimum exercise loading apparatus useful in the field of preventive medicine, rehabilitation medicine or the like, and more particularly to an optimum exercise loading apparatus which is capable of automatically providing a human being, particularly, a patient with optimum exercise load.

Description of the Prior Art

Recently, in physical therapy, there has been highly increased necessity for a patient whose lung is decreased in function to undergo training for rehabilitation, in particular, an exercise cure and respiratory training. Also, it is known in the art that an exercise cure is highly effective for the therapy of a heart disease, diabetes, corpulence and the like, In order to increase the efficiency of the exercise cure in such therapy to effectively accomplish the rehabilitation of a patient, it is highly required to determine the amount of optimum exercise load depending upon the conditions of an individual patient. Conventionally, the determination of such optimum exercise load is carried out according to an empirical and inductive method on the basis of data on the cardiogram, oxygen intake and/or carbon dioxide discharge of a patient obtained by subjecting the patient to loading exercise.

However, such a conventional procedure of determining optimum exercise load must subject a patient to loading exercise test for several days. This causes an equal amount of loading exercise to be excessive or insufficient to the patient depending upon the conditions of the patient which are varied every day. The excessive loading exercise causes the patient to get into danger and the insufficient loading exercise fails to determine optimum exercise load. Thus, it will be noted that the prior art fails to provide an optimum exercise loading apparatus which not only accomplishes the automatic and real-time determination of optimum exercise load for an individual patient and allows a patient to carry out loading exercise under the optimum conditions but effectively carries out both satisfied risk management and a satisfied positive exercise cure.

Accordingly, it is highly desirable to develop an optimum exercise loading apparatus which effectively meet such requirements as described above.

## SUMMARY OF THE INVENTION

The present invention has been made in view of the foregoing disadvantage of the prior art.

Generally speaking, in accordance with the present invention, an optimum exercise loading apparatus is provided which comprises a load section for causing a patient to carry out exercise under load; a measuring and analyzing section for measuring and analyzing at least one variable varied in the patient due to exercise of the patient at the load section during the exercise; and a control section for carrying out the comparison between a measured value of the variable obtained in the measuring and analyzing section and a target value of the variable to judge the propriety of load applied to the patient at the load section to carry out the feedback control of the load.

In a preferred embodiment of the present invention, an electrically driven treadmil is used for the load section. Alternatively, an ergometer for a bicycle, a hand-ergometer or the like may be used for the load section. For example, when a treadmil is used, the load applied to a patient at the load section may be varied by continuously or intermittently varying at least one of rotating speed and inclination angle of the treadmil. In the measuring and analyzing section, at least one of variables varied in the patient due to the exercise carried out at the load section during the exercise such as oxygen intake (hereinafter referred to also as "$VO_2$"), carbon dioxide discharge ($VCO_2$), respiratory quotient (RQ), oxygen intake/weight ($VO_2/W$), air ventilation (VE), oxygen intake/carbon dioxide discharge ($VO_2/VCO_2$), oxygen concentration in exhalation ($DO_2$) and METS ($O_2$ ml/kg/min) which are measured from the exhalation of the patient is detected. The term "METS" used herein is metabolic 10 equivalents derived from metabolite and indicates the amount of oxygen required to keep the resting state of a healthy adult subject. More particularly,

$$1 \text{ METS } = 3.5\text{mL of } VO_2 \text{ kg}^{-1} \text{ min}^{-1}.$$

Also, heart rate obtained from an electrocardiogram, a blood-sugar level or the like may be used as a variable in the present invention, other than the above-described variables which are measured from the exhalation of a patient.

The control section includes a comparator to which a microcomputer is attached. In the control section, a target value of at least one of the variables described above is set as risk restriction by a doctor or a physical therapist, and the comparison between the target value of the variable and a measured value thereof is electrically carried out. Based on the comparison, a control circuit in the load section is operated to vary the load of the treadmil or the like.

Such construction allows the state or conditions of a patient to be effectively judged from the measured value of the variable and the propriety of exercise load applied to the patient to be appropriately judged from the comparison between the measured value of the variable and the target value thereof. Load applied to the patient at the load section is varied depending upon a result of the comparison, to thereby carry out the real-time setting of optimum exercise load for the patient. This results in the patient carrying out optimum maximum loading exercise while ensuring the safety of the patient and undergoing risk management and a positive exercise cure.

In accordance with the present invention, there is also provided an optimum exercise loading apparatus comprising a load section comprising a treadmil, the load section causing a patient to carry out exercise under load which is varied by varying at least one of rotating speed and inclination angle of the treadmil; a measuring and analyzing section comprising a respiratory metabolism measuring device for analyzing and measuring $VO_2$ from the exhalation of the patient due to the exercise of the patient at the load section during the exercise; and a control section comprising a comparator, a data processing block connected to the comparator and comprising a $VO_2$ integrating circuit for integrating the measured value of the $VO_2$ obtained in the measuring and analyzing section and supplying the so-integrated $VO_2$ value to the comparator and a $VO_2$ converting circuit for converting the integrated $VO_2$ value into METS and supplying the so-converted METS to said comparator, a target block comprising a $VO_2$ target value setting circuit for supplying the target value of risk restriction for the patient to the comparator and a target METS value setting circuit, and a load control block comprising a speed and angle setting circuit for calculating the rotating speed and/or inclination angle of the treadmil based on the output of the comparator and a speed and angle control circuit for carrying out the feedback setting of appropriate rotating speed and/or inclination angle of the treadmil depending upon the calculated rotating speed and/or inclination angle of the treadmil and the current ones; the comparator carrying out the comparison between the target METS value and the converted METS value to judge the propriety of load applied to the patient at the load section to carry out the feedback control of the load.

Accordingly, it is an object of the present invention to provide an optimum exercise loading apparatus which is capable of automatically and real-timely determining the optimum exercise loading for a patient and concurrently allowing the patient to carry out loading exercise under the optimum conditions, so that both satisfied risk management and a satisfied positive exercise cure may be effectively carried out.

It is another object of the present invention to provide an optimum exercise loading apparatus which is capable of ensuring the health and safety of a patient.

It is a further object of the present invention to provide an optimum exercise loading apparatus which is capable of exhibiting many advantages in physical therapy.

It is still another object of the present invention to provide an optimum exercise loading apparatus which is capable of carrying out reliable operation.

It is yet another object of the present invention to provide an optimum exercise loading apparatus which is capable of carrying out the above-described objects with a simple structure.

Still other objects and advantages of the present invention will in part be obvious and will in part be apparent from the specification.

The invention accordingly comprises the features of construction, combination of elements, and arrangement of parts which will be exemplified in the construction hereinafter set forth, and the scope of the invention will be indicated in the claims.

BRIEF DESCRIPTION OF THE DRAWINGS

For a fuller understanding of the invention, reference is had to the following description taken in connection with the accompanying drawings in which like reference numerals designate like or corresponding parts throughout, wherein:

Fig. 1 is a diagrammatic view showing the concept of an embodiment of an optimum exercise loading apparatus according to the present invention;

Fig. 2 is a graphical representation showing the relationships between the variation in time of loading exercise and a METS value; and

Fig. 3 is a graphical representation showing the relationships between the variation in time of loading exercise and a load value.

DETAILED DESCRIPTION OF THE PREFERRED EMBODIMENT

Now, an optimum exercise loading apparatus according to the present invention will be described with reference to the accompanying drawings.

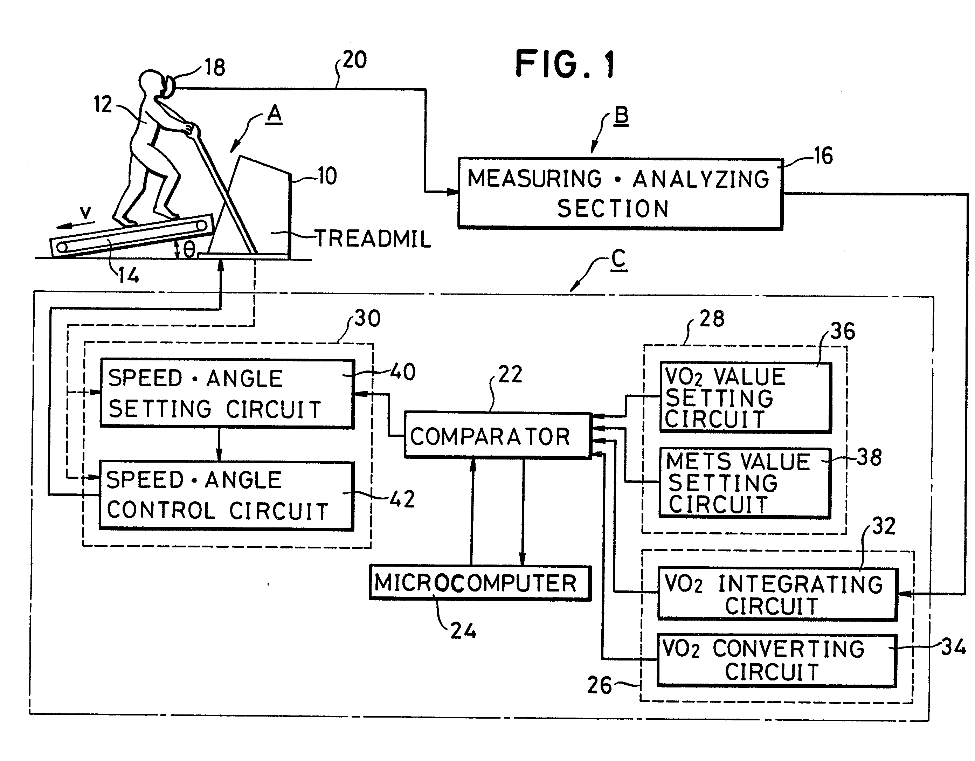

Fig. 1 schematically shows the concept of an embodiment of an optimum exercise loading apparatus according to the present invention. In Fig. 1, reference character A designates a loading section of an optimum exercise loading apparatus of the illustrated embodiment which is constituted by an electrically driven treadmil 10. The treadmil 10 is adapted to cause a patient 12 to rest or carry out walking exercise on a travelling belt 14. The travelling belt 14 is constructed to be varied in a travelling or rotating speed $v$ and an inclination angle $\theta$ as desired, so that at least one of these factors may be varied to variably set exercise load depending upon a patient.

The optimum exercise loading apparatus of the illustrated embodiment also includes a measuring and analyzing section B which comprises a device for measuring respiratory metabolism designated by reference numeral 16. In the illustrated embodiment, the respiratory metabolism measuring device 16 is adapted to gather exhalation of the patient 12 through a mask 18 held to the mouth and nose of the patient 12 and a duct 20 during the exercise and carry out the measurement and analysis of oxygen intake or $VO_2$ from the exhalation.

The optimum exercise loading apparatus of the embodiment illustrated further includes a control section C. The control section C comprises a comparator 22 having a microcomputer 24 attached thereto. The microcomputer 24 is adapted to carry out the file, statistical processing and graphic processing of individual data, or the like. For example, it carries out the fetching processing through an A-D converter to synchronize the belt speed or inclination angle of the treadmil, the heart rate of a patient, the step cycle or stance of the patient, or the like with the output of the measuring and analyzing section B.

The control section C further comprises a data processing block 26, a target block 28 and a load control block 30 which are connected to the comparator 22. The data processing block 26 includes a $VO_2$ integrating circuit 32 which serves to integrate the measured value of oxygen intake or $VO_2$ obtained in the measuring and analyzing section B and supply the so-integrated $VO_2$ value to the comparator 22 and a $VO_2$ converting circuit 34 for converting the integrated $VO_2$ value into METS and supply the so-converted METS to the comparator 22. The target block 28 includes a desired or target $VO_2$ value setting circuit 36 which is adapted to be operated through a key board (not shown) to supply the target value such as risk restriction of the patient 12 and the like to the comparator 22 and a desired or target METS value setting circuit 15. The load control block 30 includes a speed and angle setting circuit 40 for calculating the rotating speed v and inclination angle θ of the treadmil 10 based on the output of the comparator 22 and a speed and angle control circuit 42 for carrying out the feedback setting of appropriate rotating speed and inclination angle of the treadmil 10 depending upon both the rotating speed v and inclination angle $\theta$ of the treadmil 10 set or calculated by the setting circuit 40 and the current rotating speed and inclination angle. The comparator is adapted to carry out the comparison between the target METS value and the converted METS value to judge the propriety of load applied to the patient 12 at the load section A to carry out the feedback control of the load.

Now, the manner of operation of the optimum exercise loading apparatus of the illustrated embodiment constructed in the manner described above will be described with reference to the drawings.

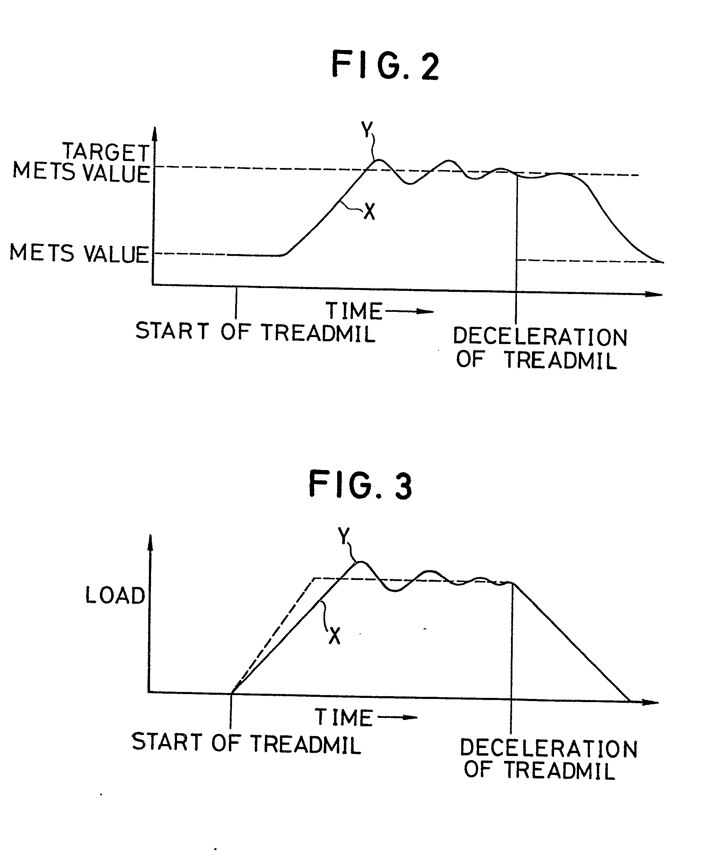

In the optimum exercise loading apparatus of the illustrated embodiment, an ideal METS value which a doctor and/or a physical therapeutist determine depending upon a patient based on the past experience is set as a desired METS value or a target value. The ideal value may be set with respect to $VO_2$. However, the following description will be made with respect to METS. The ideal METS value is fed from the target METS value setting circuit 38 to the comparator 22. Together with the target value, a METS value which was measured corresponding to the variation in time of loading exercise to take place is indicated in dotted lines in Fig. 2. Also, load applied to the patient which was varied corresponding to the variation in time of the loading exercise is indicated in dotted lines in Fig. 3.

When the treadmil 10 is driven to cause the patient 12 to start loading exercise, the exhalation of the patient is introduced into and analyzed by the respiratory metabolism measuring device 16 constituting the measuring and analyzing section B to measure $VO_2$ or oxygen intake of the patient during the exercise. Then, the so-measured $VO_2$ value is input to the control section C. In the control section C, the $VO_2$ value is first input to the $VO_2$ integrating circuit 32 to be integrated therein and then input to the $VO_2$ converting circuit 34 to be converted into

a METS value. The so-converted METS value is input to the comparator 22, in which the comparison between the target METS value and the converted or measured METS value is carried out. As a result of the comparison, the fact that the target METS value is larger than the measured target value indicates that the patient 12 leaves a margin at that time, so that the speed and angle setting circuit 40 and speed and angle control circuit 42 may be operated to gradually increase the rotating speed v and/or inclination angle θ of the treadmil 10. This causes the load applied to the patient 12 to be gradually increased as indicated by a solid line portion X in Fig. 3 and therefore the converted METS value is measured in a manner to be increased as indicated by a solid line portion X in Fig. 2. Then, when the converted or measured METS value exceeds the target METS value, the speed and angle setting circuit 40 and speed and angle control circuit 42 are reversely operated to gradually decrease the rotating speed v and inclination angle θ of the treadmil 10 to decrease the load as indicated by a solid line portion Y in Fig. 3. This causes the converted or measured METS value to be also decreased as shown by a solid line portion Y in Fig. 2. Such operation is repeated to automatically carry out the control of load so as to permit the measured METS value to substantially coincide with the target METS value. This results in the patient 12 undergoing a positive exercise cure under adequate load while efficiently ensuring the safety of the patient under safe risk management based on METS.

The control of load of the treadmil 10 may be carried out by means of at least one of the rotating speed v and inclination angle θ of the treadmil. Also, in the illustrated embodiment, the target value is set with respect to METS, however, it is a matter of course that it may be set concerning $VO_2$, as described above. Alternatively, any other suitable variables may be employed to determine the target value. The above description does not refer to electrical and mechanical arrangements of the respective sections, because such arrangements are well-known or obvious to those skilled in the art. Also, such arrangements may be readily made by those skilled in the art utilizing various well-known techniques as desired.

Also, the illustrated embodiment is constructed in a manner such that the setting of initial and terminal accelerations or angular accelerations of the treadmil 10, and the setting of acceleration, deceleration and inclination angle of the treadmil 10 during the feedback control may be carried out as desired.

As can be seen from the foregoing, the optimum exercise loading apparatus of the present invention is adapted to carry out the comparison between the target value of a predetermined variable of a patient and the measured value thereof to carry out the feedback control of exercise load applied to the patient at the load section. Accordingly, the present invention realizes to provide an individual patient with an appropriate exercise cure under the optimum exercise loading conditions while ensuring the safety of the patient, resulting in the risk management and positive exercise cure of the patient being concurrently accomplished. Thus, it will be understood that the present invention exhibits various significant improvements in physical therapy.

Also, the optimum exercise loading apparatus of the present invention is of course applicable to not only the preventive medicine and rehabilitation medicine for respiratory illness, circulatory disease and metabolic disease such as diabetes but the promotion of health and the prevention of corpulence.

It will thus be seen that the objects set forth above, and those made apparent from the preceding description, are efficiently attained and, since certain

0176277

-11-

changes may be made in the above construction without departing from the scope of the invention, it is intended that all matter contained in the above description or shown in the accompanying drawings shall be interpreted as illustrative and not in s limiting sense.

It is also to be understood that the following claims are intended to cover all of the generic and specific features of the invention herein described, and all statements of the scope of the invention which, as a matter of language, might be said to fall therebetween.

CLAIMS

1. An optimum exercise loading apparatus comprising:

a load section (A) for causing a patient to carry out exercise under load;

a measuring and analyzing section (B) for measuring and analyzing at least one variable varied in the patient due to the exercise of the patient at said load section during the exercise; and

a control section (C) for carrying out the comparison between a measured value of said variable obtained in said measuring and analyzing section and a predetermined target value of said variable to judge the propriety of load applied to the patient at said load section to carry out the feedback control of said load.

2. An optimum exercise loading apparatus as defined in Claim 1, wherein said variable is METS of the patient.

3. An optimum exercise loading apparatus as defined in Claim 1, wherein said variable is $VO_2$ measured from the exhalation of the patient.

4. An optimum exercise loading apparatus as defined in Claim 1, wherein said load section includes a treadmil (10).

5. An optimum loading apparatus as defined in Claim 4, wherein the load applied to the patient at said load section is varied by varying at least one of rotating speed and inclination angle of said treadmil.

6. An optimum exercise loading apparatus as defined in Claim 5, wherein said measuring and analyzing section comprises a respiratory metabolism measuring device (16) for analyzing and measuring $VO_2$ from the exhalation of the patient.

7. An optimum exercise loading apparatus as defined in Claim 6, wherein said control section comprises:

-13-

a comparator (22);

a data processing block (26) connected to said comparator and comprising a $VO_2$ integrating circuit (32) for integrating the measured value of said $VO_2$ and supplying the so-integrated $VO_2$ value to said comparator and a $VO_2$ converting circuit (34) for converting said integrated $VO_2$ value into METS and supplying the so-converted METS to said comparator;

a target block (28) comprising a $VO_2$ target value setting circuit (36) for supplying the target value of risk restriction for the patient to said comparator and a target METS value setting circuit (38); and

a load control block (30) comprising a speed and angle setting circuit (40) for calculating the rotating speed and inclination angle of said treadmil based on the output of said comparator and a speed and angle control circuit (42) for carrying out the feedback setting of appropriate rotating speed and/or inclination angle of said treadmil in view of the calculated rotating speed and inclination angle of said treadmil and the current ones;

said comparator carrying out the comparison between the converted METS value and the target METS value to judge the propriety of load applied to the patient at said load section to carry out the feedback control of said load.

8. An optimum exercise loading apparatus comprising:

a load section (A) comprising a treadmil (10), said load section causing a patient to carry out exercise under load which is varied by varying at least one of rotating speed and inclination angle of said treadmil;

a measuring and analyzing section (B) comprising a respiratory metabolism measuring device (16) for analyzing and measuring $VO_2$ from the exhalation of the patient due to the exercise of the patient at said load section during the exercise; and

-14-

a control section (C) comprising a comparator (22), a data processing block (26) connected to said comparator and comprising a $VO_2$ integrating circuit (32) for integrating the measured value of said $VO_2$ obtained in said measuring and analyzing section and supplying the so-integrated $VO_2$ value to said comparator and a $VO_2$ converting circuit (34) for converting said integrated $VO_2$ value into METS and supplying the so-converted METS to said comparator, a target block (28) comprising a $VO_2$ target value setting circuit (36) for supplying the target value of risk restriction for the patient to said comparator and a target METS value setting circuit (38), and a load control block (30) comprising a speed and angle setting circuit (40) for calculating the rotating speed and/or inclination angle of said treadmil based on the output of said comparator and a speed and angle control circuit (42) for carrying out the feedback setting of appropriate rotating speed and/or inclination angle of said treadmil in view of the calculated rotating speed and inclination angle of said treadmil and the current ones;

said comparator carrying out the comparison between the target METS value and the converted METS value to judge the propriety of load applied to the patient at said load section to carry out the feedback control of said load.

# FIG. 1

# FIG. 2

# FIG. 3